Europäisches Patentamt

(10) European Patent Office

Office européen des brevets

(11) Publication number: **0 196 233**
**B1**

(12) **EUROPEAN PATENT SPECIFICATION**

(45) Date of publication of patent specification: **29.08.90**

(51) Int. Cl.⁵: **A 21 D 8/04,** A 21 D 6/00, C 12 N 1/18

(21) Application number: **86302275.2**

(22) Date of filing: **26.03.86**

(54) Freeze resistant dough and novel microorganism for use therein.

(30) Priority: **27.03.85 JP 62735/85**
**26.06.85 JP 140052/85**
**02.10.85 JP 219923/85**

(43) Date of publication of application:
**01.10.86 Bulletin 86/40**

(45) Publication of the grant of the patent:
**29.08.90 Bulletin 90/35**

(84) Designated Contracting States:
**DE FR GB**

(56) References cited:
**EP-A-0 078 182**
**GB-A-2 139 473**
**US-A-3 394 008**

**Die Hefen, vol. 1, 1960 Hans Carl Nürnberg**
**Verlag "Die Hefen in der Wissenschaft"**
**Japanese Patents Report, Section Ch, vol. 84,**
**no. 48, January 4, 1985 Derwent Publications**
**Ltd. London**

(73) Proprietor: **KYOWA HAKKO KOGYO CO., LTD.**
**6-1, Ohte-Machi 1-chome**
**Chiyoda-ku Tokyo-to (JP)**

(72) Inventor: **Uno, Kazuo**
**2746-4, Naruse**
**Machida-shi Tokyo (JP)**
Inventor: **Oda, Yuji**
**1-6-16, Asahi-machi**
**Machida-shi Tokyo (JP)**
Inventor: **Shigenori, Ota, c/o Patent Depart**
**Kyowa Hakko Kogyo Co. Ltd. 6-1, Ohtemachi**
**Itchome**
**Chiyoda-ku Tokyo (JP)**

(74) Representative: **Lambert, Hugh Richmond et al**
**D. YOUNG & CO. 10 Staple Inn**
**London, WC1V 7RD (GB)**

Courier Press, Leamington Spa, England.

# EP 0 196 233 B1

## Description

The present invention relates to dough mixtures containing wheat flour and water, and more especially freeze-resistant doughs, and a novel yeast for use therein.

Frozen dough is produced by the steps of mixing the ingredients, fermenting, and freezing. It is stored at about −20°C, and is proofed before baking. Ordinary bread yeasts are susceptible to damage by fermentation before freezing. Therefore, their use is restricted to the case where rich dough which contains relatively large amounts of sugar, fats and oils, eggs, dairy products, etc., is not fermented or is fermented for a short time before freezing. When the dough fermented for a short time before freezing is baked immediately after thawing and proofing, aging of the dough is insufficient and thus the flavor characteristic of bread is poor. Further, if the dough is aged after thawing, it takes a longer time for the bread making process, and the greatest advantage of frozen dough: that freshly baked bread is readily available, would be lost. Therefore, for the production of frozen dough, a yeast which is not susceptible to damage by fermentation before freezing and storing is needed.

As the bread yeasts excellent in freeze-resistance, *Saccharomyces rosei* (Japanese Published Examined Patent Application No. 25584/84), *Saccharomyces cerevisiae* FTY (Japanese Published Examined Patent Application No. 48607/84) and *Saccharomyces cerevisiae* IAM 4274 (Japanese Published Unexamined Patent Application No. 203442/84) are known. Since *Saccharomyces rosei* and *Saccharomyces cerevisiae* FTY lack maltose fermenting ability, it is difficult to apply them to lean dough. *Saccharomyces cerevisiae* IAM 4274 has maltose fermenting ability, but its freeze-resistance in lean dough is insufficient.

In addition, *Saccharomyces rosei* is smaller in cell diameter as compared with ordinary bread yeasts, and thus has a defect that it takes a longer time to separate, wash and dehydrate the yeast in the production process.

In accordance with the present invention a bread yeast has been discovered having maltose fermenting ability and therefore applicable to lean dough and which also has excellent freeze-resistance.

In one aspect, the present invention provides a dough containing wheat flour, water and a microorganism belonging to the genus *Saccharomyces*, wherein the microorganism is *Saccharomyces cerevisiae* KYF 110 (Ferm BP-972) having a sporulation ratio of 10% or above and a trehalose content in the microbial cells of 5% or above when measured by the following methods:

### (A) Measurement of the sporulation ratio

One loopful of the microbial cells taken from an active slant is inoculated into 3.0 ml of the YPD medium (2% glucose, 1% yeast extract, 2% peptone, pH 5.0) in a 25 ml test tube, and then cultured with shaking (240 rpm) at 28°C for a day. Thereafter, the cultured microbial cells are inoculated into 3.0 ml of YPA medium (1% potassium acetate, 0.5% yeast extract, 1% peptone, pH 7.0) in a 25 ml test tube to a concentration of about $10^7$ cells/ml, and cultured with shaking (240 rpm) at 28°C for 4 days.

The obtained culture liquor is appropriately diluted, and the numbers of asci and vegetative cells present per unit section wherein more than 200 asci and vegetative cells in total are present are counted with a Thoma's hemocyte counter. The sporulation ratio is defined as the proportion of the number of asci to the total number of the asci and vegetative cells.

### (B) Measurement of the trehalose content in the microbial cells

One loopful of the microbial cells taken from an active slant is inoculated into 3.0 ml of YPD medium in a 25 ml test tube, and then cultured at 28°C for a day. Thereafter, the whole amount of the culture liquor is inoculated into 50 ml of YPD medium in a 300 ml Erlenmeyer flask and cultured with shaking (180 rpm) at 28°C for a day. The collected microbial cells are washed twice with water and pressed. Three hundred milligrams of the pressed product is placed in a 300 ml Erlenmeyer flask containing 50 ml of 10% trichloroacetic acid, and extracted at 30°C for an hour while shaking (180 rpm). The extract is centrifuged (3000 rpm, 10 minutes) to obtain a supernatant. The trehalose content in the microbial cells is determined by quantitatively analyzing the total sugar in the supernatant calculated as trehalose by Anthrone method and calculating the percentage based on the weight of the dried microbial cells.

Also provided in accordance with this invention is a novel yeast: *Saccharomyces cerevisae* KYF110 and which is specifically preferred for use in the above method. This strain has been deposited under the terms of the Budapest Treaty with the Fermentation Research Institute Agency of Industrial Science and Technology, Japan, under deposit No. FERM BP-972, by transfer from deposit No. FERM P-8162, deposited 22nd March, 1985. The mycological properties of this strain are as follows:

### A. Morphological properties

| | | |
|---|---|---|
| a. | Shape: | Short oval shape |
| b. | Size: | 3.8−5.2×4.3−6.5 micrometer |
| c. | Formation of spores: | present |
| d. | Formation of pseudomycelia: | present |
| e. | Method of multiplication: | budding |

2

B. Cultural properties
    Formation of films:                absent

C. Physiological properties
    a. Fermentation and assimilation of carbohydrates

| Carbohydrate | Fermentation | Assimilation |
|---|---|---|
| Glucose | + | + |
| Galactose | + | + |
| Sucrose | + | + |
| Maltose | + | + |
| Raffinose | + | + |
| Melibiose | – | – |
| Lactose | – | – |

    b. Assimilation of nitrates and ethylamine: absent

The above-described microbial cells and microbial cells obtained by single colony separation from commercially available bread yeasts were examined with respect to the sporulation ratio and the trehalose content according to the above-described methods. The results are given in Table 1.

EP 0 196 233 B1

TABLE 1

| Strain | Sporulation ratio (%) | Trehalose content in the microbial cells (%) |
|---|---|---|
| KYF110 | 27.8 | 7.1 |
| Commercially available Product A | 0 | 3.9 |
| Commercially available Product B | 0 | 2.4 |
| Commercially available Product C (for freezing) | 0 | 2.6 |
| Commercially available Product D | 0 | 3.1 |
| Commercially available Product E | 0 | 2.3 |
| Commercially available Product F (for freezing) | 0 | 0.8 |
| Commercially available Product G | 0 | 2.5 |
| Commercially available Product H (for freezing) | 0 | 2.4 |
| Commercially available Product I | 0 | 2.4 |
| Commercially available Product J | 0 | 2.6 |
| Commercially available Product K | 0 | 1.2 |
| Commercially available Product L | 2.0 | 1.1 |

Commercially available products in Table 1 are bread yeasts of *Saccharomyces cerevisiae*.

Next, the process for preparing a dried yeast is hereinafter described.

For example, a yeast is cultured in a medium containing a carbon source (molasses, glucose, etc.), a nitrogen source (ammonium sulfate, ammonium chloride, urea, etc.) and a phosphoric acid source (ammonium phosphate, potassium phosphate, etc.). Then, the microbial cells are separated from the culture liquor, and washed with water to obtain a suspended yeast. Further, dehydration and shaping are conducted to obtain a solid yeast.

When the suspended yeast is dried by vacuum drying, freeze-drying or by using a spray drier, it is desired to add a protein (albumin, casein, etc.), carbohydrate (glucose, maltose, fructose, sucrose, etc.), and an amino acid (glutamic acid, etc.) as a protective agent for the yeast. When the solid yeast is dried, it is desired to make the yeast into granules using an extruder equipped with wire gauze or a pelleter prior to drying. Drying is preferably conducted for a short time at as low temperature as possible, and in general, it is conducted to 40—60°C for 60—160 minutes to make the water content not greater than 6%.

The dough of the present invention may be applied not only to a variety of bread dough for white bread, pastries, French bread, raisin bread, etc., but also to Chinese steam bread (Chuka manju), yeast doughnuts, etc.

For example, frozen bread dough for white bread is generally produced by steps comprising: dough formulating → kneading → prefermentation → main kneading → floor time → dividing → rounding → fermentation → shaping → freezing.

4

As the composition for the dough formulation, wheat flour, sugar, table salt, shortening oil, yeast food, yeast, water, etc., are used. The amount of the yeast, i.e., the microorganism belonging to the genus *Saccharomyces* heretofore described, used based on the flour is generally 1—5% by weight.

Freezing is generally conducted at atmospheric pressure at −15 to −85°C. The bread produced by freezing the dough of the present invention and subsequently processing the dough in the conventional manner is superior to the bread produced using frozen bread dough containing the conventional bread yeast in appearance, specific volume, internal phase and flavor.

Examples of the present invention are given hereinafter.

Example 1

KYF110 was cultured in a 5 l-jar fermenter using molasses, and after centrifugation, microbial cells were washed and pressed. The pressed microbial cells were then used for producing white bread according to the formulation shown in Table 2 and the process shown in Table 3. The results are shown in Table 4.

TABLE 2

| Ingredients | % | Weight (g) |
|---|---|---|
| Wheat flour (high-gluten) | 100 | 800 |
| Yeast | 3 | 24 |
| Sugar | 5 | 40 |
| Table salt | 2 | 16 |
| Shortening | 5 | 40 |
| Yeast food | 0.15 | 1.2 |
| Water | 67 | 536 |

TABLE 3

| Mixing<br>Kneading temp. | L 3 min., ML 6 min., MH 5 min.<br>Non-frozen group: 28°C<br>Frozen group: 20°C | |
|---|---|---|
| Floor time | 20 min. | |
| Bench time | 15 min. | |
| | Non-frozen<br>group | Frozen group |
| Freezing | — | −80°C |
| Storage | — | −20°C, 1 & 2 months |
| Thawing | — | 30°C, 90 min. |
| Proofing | 40°C, RH85%, 1.5 cm above the baking pan | |
| Baking | 210°C, 23 min. | |

TABLE 4

| | Dough of the invention | | | Control 1 | | | Control 2 | | |
|---|---|---|---|---|---|---|---|---|---|
| | Non-frozen group | Frozen group 1[1] | Frozen group 2[2] | Non-frozen group | Frozen group 1 | Frozen group 2 | Non-frozen group | Frozen group 1 | Frozen group 2 |
| Proofing time (min.) | 53 | 82 | 95 | 44 | >150 | >180 | 58 | 107 | 135 |
| Specific volume of bread | 5.50 | 5.02 | 4.55 | 5.58 | 2.77 | —[3] | 5.57 | 4.65 | 4.29 |

Notes)
Control 1: Dough prepared using a commercially available bread yeast for ordinary dough
Control 2: Dough prepared using a commercially available bread yeast for frozen dough
[1]: Stored for 1 month
[2]: Stored for 2 months
[3]: Sample does not reach 1.5 cm above the baking pan

EP 0 196 233 B1

# EP 0 196 233 B1

As is apparent from Table 4, the dough of the present invention requires a shorter proofing time and gives a bread of a greater specific volume as compared with the control samples.

Example 2

Bread was produced according to the formulation shown in Table 5 and the process shown in Table 6 using pressed microbial cells obtained in the similar manner as in Example 1.

The results are given in Table 7.

TABLE 5

| Ingredients | Sponge mixing | | Main mixing | |
|---|---|---|---|---|
| | % | Weight (g) | % | Weight (g) |
| Wheat flour (high-gluten) | 70 | 1120 | 30 | 480 |
| Yeast | 3 | 48 | — | — |
| Sugar | 5 | 80 | 20 | 320 |
| Table salt | — | — | 1 | 16 |
| Shortening | — | — | 5 | 80 |
| Yeast food | 0.15 | 2.4 | — | — |
| Skim milk | — | — | 2 | 32 |
| Water | 40 | 640 | 22 | 352 |

TABLE 6

| Step | Sponge mixing | Main mixing | |
|---|---|---|---|
| | | Non-frozen group | Frozen group |
| Mixing | L 3 min., ML 2 min. | L 2 min., ML 6 min. | L 2 min., ML 1 min. <br> L 2 min., ML 6 min., MH 3 min. |
| Kneading | 26°C | 28°C | 20°C |
| Fermenting time | 2.5 hrs | Floor time: 20 min. <br> Bench time: 15 min. | — <br> — |
| Freezing | — | — | −80°C |
| Storage | — | — | −20°C <br> 1 month & 2 months |
| Thawing | — | — | 30°C, 90 min. |
| Proofing | — | 40°C, RH 85% 1.5 cm above the baking pan | |
| Baking | — | 210°C, 23 min. | |

7

TABLE 7

|  | Dough of the invention | | | Control 1 | | | Control 2 | | |
|---|---|---|---|---|---|---|---|---|---|
|  | Non-frozen group | Frozen group 1[*1] | Frozen group 2[*2] | Non-frozen group | Frozen group 1 | Frozen group 2 | Non-frozen group | Frozen group 1 | Frozen group 2 |
| Proofing time (min.) | 51 | 69 | 105 | 47 | 125 | >180 | 45 | 193 | >180 |
| Specific volume of bread | 5.50 | 4.92 | 4.43 | 5.55 | 3.67 | 3.47 | 5.13 | 4.33 | 3.30 |

Notes)
    Controls 1 & 2, [*1] and [*2]: Same as in Table 4.

As is apparent from Table 7, the dough of the present invention requires a shorter proofing time and gives a bread of a greater specific volume as compared with the control samples.

Example 3

(1) Low sugar dough

1 g of the dried yeast obtained in Example 5 was dissolved in 20 ml of distilled water, and the solution was added to 100 g of wheat flour together with 4 g of sugar and 1.5 g of table salt dissolved in 40 ml of distilled water. The ingredients were mixed in a mixer for 2 minutes to obtain low sugar dough. As a control, similar dough was prepared using a commercially available dried yeast instead of the dried yeast obtained in Example 5. The dough was taken out of the fixer and charged into a cylinder (diameter: 5.7 cm, height: 22 cm) designated by Yeast Kogyo Kai, and the fermenting power after 80 minutes at 30°C (first fermenting power) was measured. After molding was conducted three times using a molder, the dough was charged into the cylinder again, and the fermenting power after 50 minutes at 30°C (second fermenting power) was measured. Further, similar operations were conducted, and the fermenting power after 50 minutes at 30°C (third fermenting power) was measured. The results are shown in Table 8.

TABLE 8

| Dried yeast used | Dough (immediately after production) | | | Dough (after storage at 30°C for 1 month) | | |
|---|---|---|---|---|---|---|
| | First (ml) | Second (ml) | Third (ml) | First (ml) | Second (ml) | Third (ml) |
| Yeast obtained in Example 5 | 450 | 440 | 445 | 440 | 420 | 425 |
| Commercially available yeast | 400 | 385 | 400 | 300 | 290 | 290 |

(2) High sugar dough

1.5 g of the dried yeast obtained in Example 5 was dissolved in 20 ml of distilled water, and the solution was added to 100 g of wheat flour together with 16 g of sugar and 1.5 g of table salt dissolved in 30 ml of distilled water. The ingredients were mixed in a mixer for 2 minutes to obtain high sugar dough. As a control, similar dough was prepared using a commercially available dried yeast instead of the dried yeast obtained in Example 5. The dough was taken out of the mixer and charged into a cylinder designated by Yeast Kogyo Kai, and the fermenting power after 90 minutes at 30°C was measured. The results are given in Table 9.

TABLE 9

| Dried yeast used | Dough (immediately after production) (ml) | Dough (after storage at 30°C for 1 month) (ml) |
|---|---|---|
| Yeast obtained in Example 5 | 440 | 400 |
| Commercially available yeast | 390 | 255 |

Example 4

The bread dough ingredients shown in Table 10 were blended. As the dried yeast, the yeast obtained in Example 6 or commercially available one was used. Dough was prepared from the ingredients immediately after blending and after storage at 37°C for 1, 3 and 5 weeks, and the expanding power of the dough was measured.

TABLE 10

| Ingredients | Test group I | Test group II |
|---|---|---|
| Wheat flour (high-gluten flour) | 70 g | 70 g |
| Wheat flour (semihigh-gluten flour) | 30 | 30 |
| Sugar | 4 | 16 |
| Shortening | 4 | 7 |
| Table salt | 2 | 1.2 |
| Yeast food | 0.2 | 0.2 |
| Dried yeast | 1.5 | 2.5 |
| Distilled water | 59 ml | 46 ml |

Preparation of bread dough:

Ingredients→Mixing (2 min.)→Floor time (5 min.)→Mixing (2 min.)→Charging into cylinder (Yeast Kogyo Kai Method)→Measurement of expanding power of dough (30°C, after 60 min.: First fermentation)→Molding→Charging into cylinder→Measurement of expanding power of dough (30°C, after 60 min.: Second fermentation)

The results of the measurement of expanding power of the dough are given in Table 11.

TABLE 11

| | Test group I | | | | | | | |
|---|---|---|---|---|---|---|---|---|
| | First fermentation (week) | | | | Second fermentation (week) | | | |
| Dried yeast used | 0 | 1 | 3 | 5 | 0 | 1 | 3 | 5 |
| Yeast obtained in Example 6 | 460 ml | 420 ml | 410 ml | 390 ml | 535 ml | 510 ml | 500 ml | 490 ml |
| Commercially available yeast | 495 | 300 | 220 | 190 | 535 | 430 | 330 | 285 |

| | Test group II | | | | | | | |
|---|---|---|---|---|---|---|---|---|
| | First fermentation (week) | | | | Second fermentation (week) | | | |
| Dried yeast used | 0 | 1 | 3 | 5 | 0 | 1 | 3 | 5 |
| Yeast obtained in Example 6 | 460 ml | 430 ml | 410 ml | 390 ml | 480 ml | 460 ml | 440 ml | 410 ml |
| Commercially available yeast | 360 | 255 | 200 | 165 | 470 | 340 | 245 | 205 |

Example 5

KYF 110 was inoculated into a medium containing 11.7% molasses, 0.4% ammonium sulfate, 0.4% urea and 0.06% ammonium primary phosphate in a 5 l-jar fermenter, and cultured at pH 5.0 with aeration (6 l/min.) and stirring (600 rpm) at 30°C for 24 hours. After the completion of culturing, centrifugation was conducted (3000 rpm), and the collected cells were suspended in water, and centrifuged again. Thereafter, similar treatments were repeated twice, and the cells were pressed with a small filter press to obtain a pressed yeast. The yeast was cooled (0°C±2°C), and then extruded through a 0.7×0.7 mm wire gauze to form granules which were adjusted in size with a 3.0×3.0 mm wire gauze, and then dried using a fluidized layer drier (blowing of hot air: 15 m³/min.) at 45°C for 15 minutes.

Example 6

KYF 110 was inoculated into a medium containing 11.7% molasses, 0.4% ammonium sulfate, 0.2% ammonium chloride, 0.4% urea and 0.06% ammonium primary phosphate in a 30 l-jar fermenter, and cultured in the same manner as in Example 5. After the completion of culturing, centrifugation was conducted (3000 rpm), and the collected cells were suspended in water, and centrifuged again. Thereafter, similar treatments were repeated twice, and a yeast cake was obtained using a vacuum dehydrater. The cake was cooled (0°C±2°C), and then extruded through a 0.7×0.7 mm wire gauze. The obtained granules were dried using a shelf drier at 55°C for 70 minutes to obtain a dried yeast.

**Claims**

1. A freeze-resistant dough containing flour, water and a microorganism belonging to the genus *Saccharomyces*, characterised in that said microorganism is *Saccharomyces cerevisiae* KYF 110 (Ferm BP-972) having a sporulation ratio of 10% or above and a trehalose content in the microbial cells of 5% or above.

2. A dough according to claim 1, characterised in that the microorganism is present in the dough as a compressed or a dried yeast.

3. A dough according to claim 1 or claim 2, characterised in that the microorganism is present in the dough in an amount of from 1 to 5% by weight, based on the weight of the flour.

4. A dough according to any one of claims 1 to 3, characterised in that the flour is wheat flour.

5. A yeast of the species *Saccharomyces cerevisiae*, characterised in that the yeast is *Saccharomyces cerevisiae* KYF 110 (Ferm BP-972) having a sporulation ratio of 10% or above and a trehalose content in the microbial cells of 5% or above.

**Patentansprüche**

1. Frostbeständiger Teig, enthaltend Mehl, Wasser und einen zur Gattung *Saccharomyces* gehörenden Mikroorganismus, dadurch gekennzeichnet, daß der Mikroorganismus *Saccharomyces cerevisiae* KYF 110 (Ferm BP-972) ist, welcher ein Sporulierungsverhältnis von mindestens 10% und einen Trehalose-Gehalt in den Mikrobenzellen von mindestens 5% aufweist.

2. Teig nach Anspruch 1, dadurch gekennzeichnet, daß der Mikroorganismus in dem Teig als Preß- oder Trockenhefe vorhanden ist.

3. Teig nach Anspruch 1 oder Anspruch 2, dadurch gekennzeichnet, daß der Mikroorganismus in dem Teig in einer Menge von 1 bis 5 Gew.-%, bezogen auf das Gewicht des Mehls, vorhanden ist.

4. Teig nach einem der Ansprüche 1 bis 3, dadurch gekennzeichnet, daß das Mehl Weizenmehl ist.

5. Hefe der Art *Saccharomyces cerevisiae*, dadurch gekennzeichnet, daß die Hefe *Saccharomyces cerevisiae* KYF 110 (Ferm BP-972) ist, welche ein Sporulierungsverhältnis von mindestens 10% und einen Trehalosegehalt in den Mikrobenzellen von mindestens 5% aufweist.

**Revendications**

1. Pâte résistant à la congélation, qui contient de la farine, de l'eau et un microorganisme appartenant au genre *Saccharomyces*, caractérisée en ce que ledit microorganisme est *Saccharomyces cerevisiae* KYF 110 (Ferm BP-972) ayant un taux de sporulation de 10% ou plus et une teneur en tréhalose dans les cellules microbiennes de 5% ou plus.

2. Pâte conforme à la revendication 1, caractérisée en ce que le microorganisme est présent dans la pâte sous forme de levure comprimée ou sèche.

3. Pâte conforme à la revendication 1 ou 2, caractérisée en ce que le microorganisme est présent dans la pâte à raison de 1 à 5% en poids par rapport au poids de la farine.

4. Pâte conforme à l'une quelconque des revendications 1 à 3, caractérisée en ce que la farine est de la farine de blé.

5. Levure de l'espèce ‛*Saccharomyces cerevisiae*, caractérisée en ce que ladite levure est *Saccharomyces cerevisiae* KYF 110 (Ferm BP-972) ayant un taux de sporulation de 10% ou plus et une teneur en tréhalose dans les cellules microbiennes de 5% ou plus.